# EUROPEAN PATENT SPECIFICATION

(11) **EP 0 294 028 B1**
(45) Date of publication and mention of the grant of the patent: **01.09.1993**
(21) Application number: 88303930.7
(22) Date of filing: 29.04.1988
(51) Int. Cl.: A61K 31/135

(54) **Fluoxetine useful for the treatment of diabetes**
Verwendung von Fluoxetin zur Behandlung des Diabetes
L'emploi de fluoxétine pour le traitement du diabète

(30) Priority: 04.05.1987 US 45509
(43) Date of publication of application: 07.12.1988
(73) Proprietor: ELI LILLY AND COMPANY, Indianapolis, Indiana 46285 (US)
(72) Inventor: Wong, David Taiwai, Indianapolis Indiana 46217 (US)
(74) Representative: Tapping, Kenneth George

(56) References cited:
- US-A- 4 626 549
- INT. J. OBES., vol. 11, no. Suppl. 3, 15th December 1987, pages 163-170; J.M. FERGUSON and J.P. FEIGHNER: "Fluoxetine-induced weight loss in overweight non-depressed humans"
- EUR. J. PHARMACOL., vol. 78, no. 3, 1982, pages 263-270; G.A. WILSON and B.L. FURMAN: "Effects of inhibitors of 5-hydroxytryptamine uptake on plasma glucose and their interaction with 5-hydroxytryptophan in producing hypoglycaemia in mice"
- CURR. MED. RES. OPIN., vol. 6, no. suppl. 1, 1979, pages 28-33; J.J. WURTMAN and R.J. WURTMAN: "Fenfluramine and other serotoninergic drugs depress food intake and carbohydrate consumption while sparing protein consumption"
- DIABETOLOGIA, vol. 32, no. 7, 1989, page 557A; S.D. WISE: "Fluoxetine, efficacy and safety in treatment of obese Type 2(non-insulin-dependent) diabetes"

## Description

This invention belongs to the field of clinical medicine, and provides a new method of controlling diabetes by administering fluoxetine to the patient.

The control of diabetes has long been one of the primary concerns of medical research. While the discovery of convenient and economical methods for obtaining insulin from animal sources, and more recently, synthesizing human insulin, has brought about a revolution in the control of diabetes, other methods of treating diabetes are still of great concern. The necessity to administer insulin by percutaneous means results in complexities, and even danger, in administration. Thus, pharmaceutical methods of controlling diabetes, particularly with orally-administered drugs, are valuable and are in demand.

Fluoxetine is now a very well known drug. Its chemical name is 3-(4-trifluoromethylphenoxy)-N-methyl-3-phenylpropylamine. It was described in U.S. Patent 4,018,895, and was shown to be useful in treating depression. It has since been found to be useful for a surprising variety of other conditions, which are described in U.S. Patents 4,194,009, 4,329,356, 4,590,213, 4,594,358, 4,626,549 and 4,647,591.

In general, diabetic patients are divided into two groups, Type I and Type II. Type I diabetics are those who require insulin for the preservation of life. Type II diabetics, the more numerous class, are those who may require insulin or oral hypoglycemics to control their elevated blood sugars. Type II diabetics usually become diabetic as adults and frequently are overweight and have poor dietary habits.

This invention provides a method for controlling diabetes in mammals which comprises administering to a diabetic, in which fluoxetine does not cause weight loss, an effective dose of fluoxetine or a pharmaceutically acceptable salt thereof.

The method of this invention is carried out by administering a pharmaceutical formulation of fluoxetine or a pharmaceutically acceptable salt thereof to a mammal in need of such treatment. Formulations of fluoxetine and its salts are described in U.S. Patent 4,194,009. The nature and preparation of pharmaceutically acceptable salts of fluoxetine are explained in detail in that patent, as well as in U.S. Patent 4,329,356. The preferred salts are those prepared with mineral acids, especially the hydrochloride salt.

One of the advantages of fluoxetine is that it can be orally administered. Thus, the preferred route of administration of the drug is orally, in the usual pharmaceutical forms such as tablets, capsules, and suspensions However, the drug and its salts are equally effectively administered by subcutaneous, intravenous, intramuscular and rectal routes and may be administered in such ways if it is convenient in a given instance.

Of course, the preferred patients to which the method of the present invention is to be applied are diabetic humans. However, a number of animal species are subject to diabetes, and the method may be applied to any such species within the concept of the present invention.

Fluoxetine is effective over a wide dosage range. Most preferably, when the method is applied to diabetic humans, doses in the range from 20 mg/day to 90 mg/day are administered. Even more preferable are doses from 40 mg/day to 80 mg/day. In general, however, doses of fluoxetine or its salts in the range from 0.1 mg/kg/day to 50 mg/kg/day, more preferably in the range from 0.2 to 10 mg/kg/day, may usefully be administered.

It is clear that fluoxetine is effective when administered as the only anti-diabetic agent. However, it may also be usefully administered in combination with insulin or with other hypoglycemic drugs, such as sulfonylureas. When such combination therapy is practiced, the attending physician must determine the proper doses of fluoxetine and the other agent. In general, however, the dose of fluoxetine will be in the range described above, and the dose of the other agent will also be in the usual range for that therapeutic agent.

The effect of the present invention in controlling diabetes has been shown in a controlled clinical experiment under the management of qualified medical investigators. In this experiment, 66 obese Type II diabetic patients were administered 60 mg of fluoxetine hydrochloride per day, in tablet form. A group of 67 similar patients were administered placebo tablets in a double-blind experiment.

The length of the experiment was 56 days. Blood samples were taken from all patients at the beginning and end of the experiment, and at intervals within the experiment. Various analyses were done on the blood samples; those of particular interest from the standpoint of the control of diabetes are the fasting blood glucose level, and the hemoglobin A1c level. The level of blood glucose indicates the total amount of glucose in the blood, and is a direct indication of the quality of control of diabetes obtained. Hemoglobin A1c measures the amount of glucose bound to hemoglobin, and is a measure of the average control of diabetes obtained over the 2-3 months previous to the analysis.

The method for determining blood glucose was that of Bondar and Mead, Clin. Chem. 20, 256 (1974), and the hemoglobin Alc method was that of Goldstein et al., Clin. Chem. 32, (B), B64 (1986).

All patients were weighed each time that they were seen by the investigators.

Fluoxetine is known to control obesity (U.S. Patent 4,626,549), and the fluoxetine-treated patients as a group lost weight, as expected. The mean weight loss of patients on placebo was 0,5897 kg (1.3 pounds), and the mean weight loss of the fluoxetine-treated patients was 2,6672 kg (5.88 pounds). The difference in weight loss was significant at the P<.001 level.

However, 12 fluoxetine-treated patients did not experience the expected weight loss. Some gained weight, and some lost a very small amount, less than the placebo patients lost. Those 12 patients, however, exhibited control of their diabetes which was not associated with weight loss.

The mean decline in fasting blood glucose of the placebo patients was, at most, 5 mg/100 ml of blood. The mean decrease in fasting blood glucose level in the 12 fluoxetine-treated patients was 54 mg/100 ml. The difference in effect between the 12 patients and the placebo group was statistically significant at the P = 0.033 level.

For various reasons, hemoglobin Alc data was not obtained for three of the 12 fluoxetine-treated patients who did not lose weight. Five of the other nine of them exhibited an absolute decline in their hemoglobulin A1c concentrations which ranged from 1.5% to 4.5%. The corresponding levels in the placebo patients declined by less than 0.5%. (These decreases are to be compared with the typical concentrations in stable diabetics, from 4.2% to 11.2%.) The hemoglobulin A1c data has been statistically analyzed and the difference between the nine fluoxetine-treated patients and the placebo group is significant at the level P = 0.037.

## Claims

1. The use of fluoxetine or a physiologically acceptable salt thereof for the manufacture of a medicament for the treatment of diabetes in a diabetic patient without causing loss of weight.

2. A use of claim 1 in which the patient is a Type II diabetic.

3. A use of claim 1 or 2 in which the medicament is orally administered.

4. A use of claim 1, 2 or 3 in which fluoxetine is in the form of a mineral acid salt.

5. A use of claim 1, 2 or 4 in which the amount of fluoxetine is from 20 to 90 mg/day.

6. A use of claim 5 in which the amount of fluoxetine is from 40 to 80 mg/day.

## Patentansprüche

1. Verwendung von Fluoxetin oder einem physiologisch annehmbaren Salz hiervon zur Herstellung eines Arzneimittels für die Behandlung von Diabetes bei einem Patienten mit Diabetes ohne Verursachung eines Gewichtsverlustes.

2. Verwendung nach Anspruch 1, worin der Patient ein Diabetiker vom Typ II ist.

3. Verwendung nach Anspruch 1 oder 2, worin das Arzneimittel oral verabreicht wird.

4. Verwendung nach Anspruch 1, 2 oder 3, worin Fluoxetin in Form eines Mineralsäuresalzes vorliegt.

5. Verwendung nach Anspruch 1, 2 oder 4, worin die Menge an Fluoxetin 20 bis 90 mg pro Tag beträgt.

6. Verwendung nach Anspruch 5, worin die Menge an Fluoxetin 40 bis 80 mg pro Tag beträgt.

## Revendications

1. Utilisation de la fluoxétine ou d'un sel pharmaceutiquement acceptable de celle-ci pour la fabrication d'un médicament pour le traitement du diabète chez un patient diabétique sans provoquer de perte de poids.

2. Utilisation selon la revendication 1, dans laquelle le patient est un diabétique de type II.

3. Utilisation selon la revendication 1 ou 2, dans laquelle le médicament est administré par voie orale.

4. Utilisation suivant la revendication 1, 2 ou 3, dans laquelle la fluoxétine se présente sous la forme d'un sel d'un acide minéral.

5. Utilisation selon la revendication 1, 2 ou 4, dans laquelle la quantité de fluoxétine est comprise entre 20 et 90 mg/jour.

6. Utilisation selon la revendication 5, dans laquelle la quantité de fluoxétine est comprise entre 40 et 80 mg/jour.
